# EUROPEAN PATENT SPECIFICATION

(11) **EP 1 153 585 B1**
(45) Date of publication and mention of the grant of the patent: **20.10.2004**
(21) Application number: 01203078.9
(22) Date of filing: 13.08.1997
(51) Int. Cl.: A61F 13/15, A61F 13/56

(54) **Disposable diaper**
Wegwerfwindel
Couche culotte

(30) Priority: 19.08.1996 JP 21737796; 19.11.1996 JP 30810896; 26.12.1996 JP 34846596
(43) Date of publication of application: 14.11.2001
(62) Divisional of application: 97306172.4
(73) Proprietor: Uni-Charm Corporation, Shikokuchuo-shi, Ehime-ken (JP)
(72) Inventor: Fujioka, Yoshihisa, Mitoyo-gun, Kagawa-ken (JP); Yamaki, Rumi, Kawanoe-shi, Ehime-ken (JP); Ono, Yoshio, Kawanoe-shi, Ehime-ken (JP)
(74) Representative: Parry, Christopher Stephen

(56) References cited:
- EP-A- 0 641 552
- EP-A- 0 758 543
- WO-A-90/04374
- WO-A-91/09580
- WO-A-95/06451
- GB-A- 2 262 873
- US-A- 4 351 340
- US-A- 4 775 375
- US-A- 5 382 246

## Description

The present invention relates to a disposable diaper which fits the body of a wearer and is designed to prevent the positional shift of a protector against incontinence and the side oozing of for example urine particularly when such protector against incontinence is used.

Disposable diaper comprises a back sheet, a top sheet and an absorption core interposed between the two sheets. The back sheet facing outward comprises a resin sheet, liquid non-permeable but finely air permeable so as to prevent the outside oozing of urine. Furthermore, the top sheet facing toward the side of skin is therefore in direct contact to skin and works as the side to be spotted with urine. Hence, the top sheet is formed of a material gentle to skin, such as non-woven fabric and soft porous sheets, both liquid permeable, so that the top sheet might be permeated with fluids such as urine into the absorption core. As the absorption core, additionally, use is made of a crushed pulp or a mixture of a crushed pulp and super absorbent polymers.

So as to fit the body outline of a wearer, the absorption core is preliminarily cut and molded for example as a sandglass-like form. After molding, the absorption core is interposed between the back sheet and the top sheet. The back sheet and the top sheet both are of a shape similar to the shape of the absorption core but with a larger outline than the absorption core. At both the ends thereof where no absorption core is present, these back sheet and top sheet are bonded together by means of a hot-melt adhesive.

The back waist region of disposable diaper is attached to the dorsal area of a wearer; the crotch region thereof is attached to the crotch thereof; and the front waist region is attached to the abdominal area thereof. By drawing forward the flaps of the back waist region which flaps elongate in the width direction of the diapers to overlay the flaps over the front waist region, the front waist region and the flaps are fastened together. Thus, the diapers are completely worn.

Generally, flat elastic braids capable of elongating toward the longitudinal direction (lengthwise direction) of the diapers are partially bonded, at their elongated state between the back sheet and the top sheet, to the end parts of the crotch region in the crosswise direction (width direction) by means of a hot-melt adhesive, whereby gathering is formed at the thigh parts of the diapers.

Disposable diaper for adults is far larger than disposable diaper for infants and toddlers, requiring a larger volume of raw materials for one diaper piece and thus costing high per unit price. Therefore, it is not economical to exchange the diapers for adults on each excretion of urine. Accordingly, a protector against incontinence is generally used in combination of the diapers. After urine excretion, then, only the protector against incontinence is to be exchanged.

Such protector against incontinence is formed of a lamination body in a sheet-like form, comprising an outer sheet comprising for example a liquid non-permeable resin sheet, an inner sheet comprising for example a non-woven fabric, and an absorption core interposed between the two sheets. At the ends in the lamination body in the width direction and the longitudinal direction where no absorption core is present, the outer sheet and the inner sheet are bonded together by means of a hot-melt adhesive. To the ends in the width direction, additionally, an elastic member such as flat elastic braids are partially bonded at its elongated state between the outer sheet and the inner sheet. The elastic member forms gathering.

The lamination body in the sheet-like form is preliminarily molded in the shape of a cone so that the center of the lamination body in the sheet-like form might be the peak when the protector against incontinence is worn. Through an adhesive part, both the ends of the lamination body are bonded together.

Disposable diaper should be used in such a manner that when a male wearer wears such protector against incontinence, the top sheet of diapers adheres to the adhesive part in the crotch region of the protector to prevent the shift of the protector in the diaper.

A protector against incontinence of the same structure may be used as a protector against incontinence for females. While the longitudinal direction of the lamination body should be aligned along the longitudinal direction of a disposable diaper, the outer sheet of the protector should face the top sheet of the disposable diaper to overlay the protector over the crotch region of the diaper. Then, the adhesive part of the protector against incontinence adheres to the top sheet of the diaper to prevent the shift of the protector on the diaper. In such manner, the protector is used.

These diapers are preliminarily molded as a sandglass-like form, wherein the width of the crotch region is narrower than the widths of the front waist region and the back waist region. Because urine is mainly absorbed in the crotch region, however, the crotch region should be at some width dimension so as to absorb urine. Therefore, the crotch region is formed at a larger width than the width of the crotch of humans.

At a state of such diaper worn, the crotch region expands in a bag-like shape because the crotch region is pressed by the thigh parts of a wearer from both the sides in the width direction, so that a space is formed between the crotch of the wearer and the diaper. When the diaper is used in combination with a protector against incontinence, therefore, the protector is positioned in the expanding part in front of the crotch region of the diaper. Then, the protector against incontinence is not pressed against the body. When the wearer makes a motion, therefore, the protector so readily makes a positional shift on the diaper that the protector cannot effectively absorb urine.

In the border region between the front waist region and the crotch region of a diaper, in particular, the individual dimensions of these regions in the width directions are prominently different from each other. When the crotch region is pressed by the thigh parts, therefore, the side of the front waist region essentially expands in the border region between the crotch region and the front waist region. The protector against incontinence for males is mounted at a position closer to the front waist region in the border region between the front waist region and the crotch region. When the front waist region expands as has been described above, therefore, the force of the diaper to press the protector is weakened especially for males, to readily cause the positional shift of the protector against incontinence.

When the diaper is directly worn without any protector against incontinence, excretion such as urine and feces might by chance ooze from the space between the diaper and the body of the wearer sideward. Additionally, excretion cannot be retained at a given position of the diaper, which may make the wearer feel unpleasant feeling. In this case, also, a part closer to the side of the front waist region in the border region between the front waist region and the back waist region mainly expands readily, so that excretion easily oozes out from the part.

GB2262873A discloses a disposable diaper comprising a liquid non-permeable back sheet, a liquid permeable top sheet and an absorption core interposed between the two sheets, having a front waist region to be applied to the abdominal area of a wearer, a back waist region to be applied to the dorsal area thereof, and a crotch region to be applied to the crotch, wherein thin thickness parts in the absorption core where either no absorption core is present or where the thickness of the absorption core is reduced are formed in a linearly elongating fashion in the longitudinal direction at least in the crotch region, provided that the direction from the front waist region through the crotch region to the back waist region is defined as the longitudinal direction; wherein elastic members are arranged to give a shrinkage force to the absorption core toward the direction of crossing the thin thickness parts. The elastic members are arranged throughout a region covering the whole of the crotch region and parts of the front and rear waist regions.

The present invention comprises a disposable diaper comprising a liquid non-permeable back sheet, a liquid permeable top sheet and an absorption core interposed between the two sheets, having a front waist region to be applied to the abdominal area of a wearer, a back waist region to be applied to the dorsal area thereof, and a crotch region to be applied to the crotch, wherein thin thickness parts in the absorption core where either no absorption core is present or where the thickness of the absorption is reduced are formed in a linearly elongating fashion in the longitudinal direction at least in the crotch region, provided that the direction from the front waist region through the crotch region to the back waist region is defined as the longitudinal direction; wherein elastic members are arranged to give a shrinkage force to the absorption core toward the direction of crossing the thin thickness parts; characterised in that the elastic members are arranged on the side of the absorption core facing the backsheet, and are arranged only in a region of the absorption core which extends only over a part of the front waist region and an adjacent part of the crotch region which is located between the centre of the diaper in the longitudinal direction and the front waist region.

When the thin thickness parts are present in both the crotch region and the front waist region, preferably, the thin thickness parts formed in the crotch region and the thin thickness parts formed in the front waist region are alternately aligned around the border between the crotch region and the front waist region and elastic members are arranged at parts where the thin thickness parts are alternately aligned to give a shrinkage force to the absorption core.

The present invention efficiently exerts its effect in a disposable diaper capable of mounting a protector against incontinence of the interior side of the top sheet. When the diaper of the present invention is used in combination with a protector against incontinence, the protector against incontinence should be mounted on the parts to arrange the elastic members.

In an embodiment of the diaper to mount the protector against incontinence, the thin thickness part elongating toward the side of the front waist region do not cross with the thin thickness parts elongating toward the side of the crotch region, at a part closer to the side of the front waist region from the center of the diaper in the longitudinal direction. A part with no thin thickness part of the adsorption core formed, namely a thick thickness part(s) where adsorption core is thick, is (are) formed at a given width along the longitudinal direction of the diaper. The protector against incontinence is mounted in the border region between the front waist region and the crotch region, and preferably, elastic members are arranged in the region.

When fastener means to overlay and fasten the back waist region onto the front waist region with the thin thickness parts formed thereon are arranged on the surface of the front waist region, the thin thickness parts formed in the front waist region preferably elongates into the region where the fastener means are arranged. When the fastener means are individually of the rectangular shape or a band-like shape with the longer side in the crosswise direction (width direction) of the diaper, preferably, the thin thickness parts are also formed in the same zone as the fastener means are arranged.

Additionally, it is preferable that the elastic members are arranged between the absorption core and the back sheet and that the elastic members, the top sheet and the back sheet adhere together to be fixed together at the thin thickness parts.

At a developed state of the diaper on a plane, the elastic members are possibly arranged so that the elastic members might form a curve toward the center of the crotch region in the longitudinal direction.

In the diaper of the present invention, the term "thin thickness part" means a part where no absorption core is present but the top sheet and the back sheet are directly bonded together by means of a hot-melt adhesive or a part where the thickness of the absorption core is thinner than at the other parts. In accordance with the present invention, the term "longitudinal direction" means the direction from the front waist region through the crotch region to the back waist region or the direction from the back waist region to the front waist region.

In accordance with the present invention linear thin thickness parts elongating toward the longitudinal direction of the diaper are formed, while elastic members giving shrinkage force toward the direction vertical to the linear thin thickness parts (crosswise direction) are arranged. The elastic members, for example flat elastic braids, are arranged in such a fashion that a plurality of the members arranged at some interval in the longitudinal direction might elongate toward the crosswise direction of the diaper.

The elastic members are arranged on the exterior side of the absorption core, to be kept at their elongating state between the absorption core and the back sheet, while the elastic members are bonded to the back sheet by means of a hot-melt adhesive. When no absorption core is present at the thin thickness parts, the elastic members are bonded to the interior side of the back sheet at the thin thickness parts and additionally to the top sheet by means of a hot-melt adhesive.

The elastic members serve to give an elastic force to shrink the absorption core and the back sheet in the crosswise direction, and thus, the absorption core and the back sheet shrink in the crosswise direction to form front gathering. Once the front gathering is formed, the front waist region does not expand in the direction free from the body when the crotch region is interposed in the crotch of a wearer, so that the diaper readily fits the crotch of the wearer.

By arranging elastic members with a larger elastic force than the rigidity of the absorption core, more specifically, the elastic members can work to give a shrinkage force toward the direction vertical to thin thickness parts. Thus, parts between the thin thickness parts are drawn together through the thin thickness parts and shrunk toward the crosswise direction, whereby the absorption core deforms to slightly protrude toward the inside of the diaper to make the diaper adhere to the body. In other words, then, the diaper hardly expands in the direction free from the body, so that the diaper readily fits the body. When the elastic members, the back sheet and the top sheet are bonded together at the thin thickness parts, in particular, the shrinkage force from the elastic members directly acts on the top sheet and the back sheet. Then, individual parts between the adjacent thin thickness parts are readily drawn together.

In accordance with the present invention the front gathering to shrink the absorption core in the crosswise direction is partially formed in the crotch region closer to the front waist region from the center region of the diaper in the longitudinal direction; otherwise, the front gathering is formed in the border region between the front waist region and the crotch region. More preferably, however, the front gathering is partially formed in the front waist region, the crotch region and the border region between the two regions (the border region between a part with a larger dimension of the sandglass-like absorption core in the crosswise direction and a part with a smaller dimension thereof). In other words, no elastic members are arranged excluding these parts (in front of the parts and behind the parts); no elastic members are arranged at least at a part of the front waist region or the crotch region. When such type of the diaper is worn on the body of males or females, the position spotted with urine resides in the side of the front waist region from the center of the diaper in the longitudinal direction. Thus, the region where the elastic members are arranged in the diaper of the present invention nearly corresponds to the position to be spotted with urine.

Because the absorption core is partially shrunk from the outer side toward the crosswise direction at the part corresponding to the position to be spotted with urine, the absorption core present at the position to be spotted with urine is pressed against the body to fit the body, to effectively prevent the outside oozing of urine. The structure of the diaper of the present invention is particularly effective for a diaper to be mounted with a protector against incontinence (supplementary pad) on the interior side of the top sheet. The position to mount the protector against incontinence in the diaper just corresponds to the position mounting the elastic members. Therefore, the front gathering can prevent the expansion of the diaper at the part for mounting the protector against incontinence. Hence, the protector against incontinence structurally adheres to the body, to prevent the positional shift of the protector against incontinence. When the elastic members are arranged in a given zone in the border region between the front waist region and the crotch region in the longitudinal direction, the front gathering can securely press the protector against incontinence against the body of any male or female.

Because the part where the elastic members are arranged resides in the part on the side of the front waist region from the center of the diaper or resides only in the border region between the front waist region and the crotch region, furthermore, the absorption core can fit the body at its natural state excluding these parts. Along the crosswise direction, an excessively large shrinkage force cannot act on the back waist region corresponding to the dorsal area, the crotch region interposed in the crotch, the parts where the retaining members are arranged in the front waist region and the back waist region. Therefore, the diaper can naturally fit the individual parts of the body. Because the area of the back waist region in the diaper is large, in particular, the back waist region can securely cover the dorsal area and can therefore fit the dorsal area. Accordingly, such diaper can effectively prevent the oozing of feces.

If the thin thickness parts elongating from the crotch region and the thin thickness parts elongating from the front waist region are alternately aligned in the border between the crotch region and the front waist region, the number of the thin thickness parts is large at the parts where the two types of the thin thickness parts are aligned. Therefore, the number of division of the absorption core through the presence of the thin thickness parts is so large that the border region between the front waist region and the crotch region receives the shrinkage force from the elastic members so that the absorption core can prominently shrink in the crosswise direction. Accordingly, the expansion of the border region between the crotch region and the front waist region in the diaper in the direction free from the body can be prevented.

Because the diaper can fit the crotch of wearer even without any protector against incontinence, additionally, the diaper can prevent side oozing of urine or feces to securely retain urine and feces on the diaper. Because the thin thickness parts linearly formed in the longitudinal direction have a function to disperse and introduce urine over the entire area of the diaper, urine can securely be absorbed in the absorption core.

Furthermore, the front zone of the crotch region or the border region between the crotch region and the front waist region, namely the region between the thin thickness parts formed in the crotch region and the thin thickness parts formed in the front waist region, does not have any thin thickness parts formed therein but may be formed as thick thickness part(s). The term "thick thickness part" means a part with a larger thickness of the absorption core than the thickness of the absorption core at the thin thickness part. Therefore, the thick thickness part(s) may satisfactorily be of the same thickness as the thickness of the other parts with no thin thickness part formed or may be thicker than the thickness of the other parts with no thin thickness part formed. Additionally, the front gathering formed by the elastic members resides in the region including the thick thickness part(s) therefore protector against incontinence mounted in the region including the thick thickness part(s). As has been described above, each parts interposed between the thin thickness parts are drawn toward the crosswise direction by means of the elastic members on the part of the front gathering, while at the parts where the thin thickness parts are formed, the recess on the side of the top sheet is increased to cause prominent irregularity. By forming at least a part as the thick thickness part(s) in the region where the front gathering is formed, the surface of the top sheet does not generate recess but is kept flat. When the adhesive means of the protector against incontinence is bonded to the thick thickness part(s), the bonding strength between the top sheet and the protector against incontinence can be increased to effectively prevent the positional shift of the protector against incontinence.

Because the thick thickness part(s) reside in the position to be spotted with urine, a large volume of urine can be absorbed in the thick thickness part(s). Even from such respect, the thick thickness part(s) with not any thin thickness parts present therein is preferably formed.

Preferably, furthermore, linear thin thickness parts are formed in the front waist region and the thin thickness parts elongate to the position of the retaining members to be arranged in the front waist region. When the diaper is worn on the body, the front waist region receives the shrinkage force in the crosswise direction. Owing to the shrinkage force, the surface of the retaining members readily deforms in a wave form. If thin thickness parts are arranged at the parts of the retaining members, however, the thin thickness parts shrink in the crosswise direction but the part between the thin thickness parts hardly shrink or hardly deforms when the shrinkage force in the crosswise direction acts on the front waist region, so that the surface of the retaining members is readily maintained to be nearly smooth. Thus, the area of the retaining members in the front waist region with which the retaining members of the back waist region are to be attached can so sufficiently be secured to improve the retaining strength. Particularly when elastic members elongating in the crosswise direction are arranged around the retaining members, the shrinkage force from the elastic members is readily given to the retaining members in the crosswise direction. Even then, a plurality of thin thickness parts are arranged on the back side of the retaining members of the front waist region, and therefore, the whole surface area of the retaining members hardly deforms in a wave form to enlarge the flat area of the retaining members capable of fastening.

If the elastic members are arranged to protrude toward the center of the longitudinal direction of the diaper from both the ends of the diaper in the crosswise direction to the center thereof, the front waist region of the diaper readily deforms depending on the shape of the crotch of a wearer to make the diaper fit the body.

Embodiments of the invention are described below with reference to the accompanying drawings, in which:
Fig. 1 is a perspective view of the diaper of the present invention at a state when the diaper is worn;
Figs.2 (A) and (B) are developed plane views of the diaper of the present invention; Fig.2(A) is a developed plane view of the front side of the diaper; and Fig.2(B) is a developed plane view of the back side thereof;
Figs.3 (A) and (B) are cross sectional views of Fig.2 or Fig.4 along line III - III; Fig.3(A) depicts the diaper at a flat state without shrinkage; Fig.3(B) depicts the diaper at a state when the elastic members shrink to form front gathering G;
Figs.4(A) and (B) are developed plane views of another example of the diaper of the present invention; Fig.4(A) is a developed plane view of the front side of the diaper; and
Fig.4(B) is a developed plane view of the back side of the diaper;
Fig.5 is a developed view of the other example of the diaper of the present invention; and
Fig.6 depicts a protector against incontinence; Fig.6(A) is a developed view; and Fig. 6 (B) is a perspective view of the protector at a state when it is used.

The present invention will be described in the following examples with reference to drawings hereinbelow.

Diaper 15 is formed of a lamination body, comprising back sheet 16 and top sheet 17, and absorption core 18 interposed between the two sheets. Because the back sheet 16 faces toward the exterior side of a body, the back sheet comprises a resin sheet, liquid non-permeable but air permeable, so as to prevent the oozing of urine outside. Because the top sheet 17 faces toward the skin side of a wearer and is therefore in direct contact to the skin, a material gentle to skin such as liquid permeable, non-woven fabric, is used for the top sheet. For example, use is made of a non-woven fabric with a base weight of 15 g/m² or more to 25 g/m² or less.

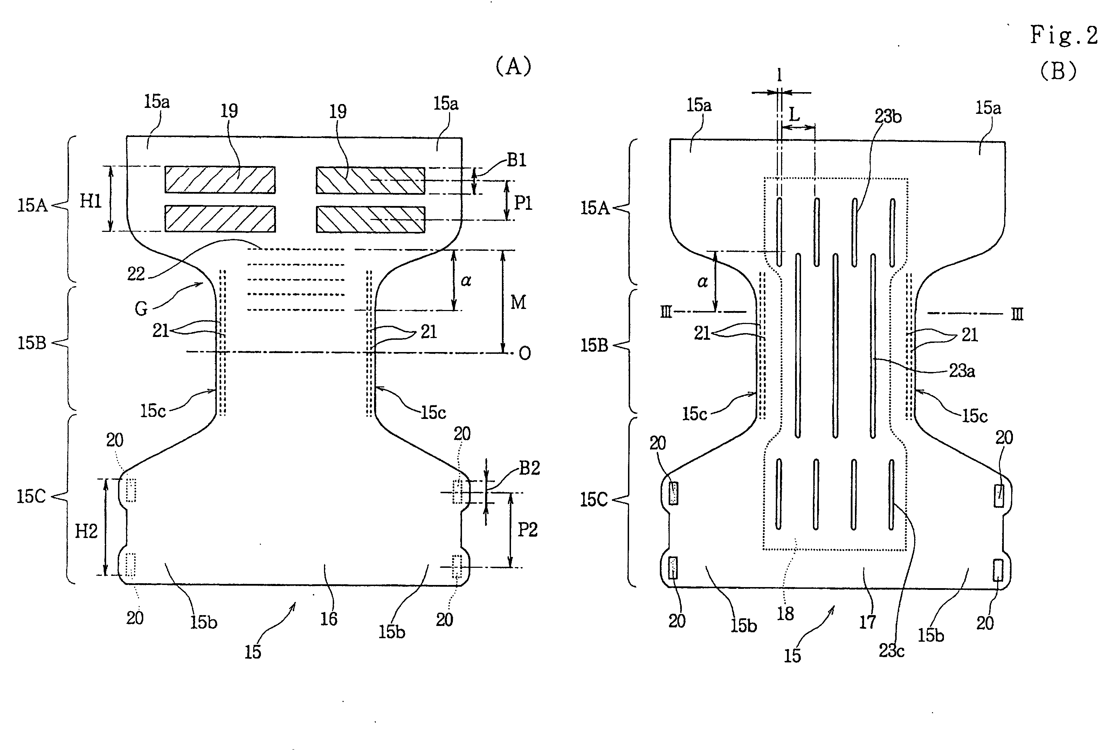

As the absorption core 18, use is made of a crushed pulp or a mixture of a crushed pulp with a super absorbent polymer, capable of well absorbing urine but never causing any unpleasant feeling for the wearer. As the crushed pulp for use as the absorption core 18, generally, use is made of a crushed pulp of a light weight with for example a specific gravity of 100 g/m² or more to 500 g/m² or less. The absorption core 18 is produced by preparing a crushed pulp or super absorbent polymers in a sheet-like form, thereafter preparing the thickness thereof uniformly and cutting the resulting sheet in a sandglass-like form with the width of crotch region 15B more or less narrower. The region surrounded by dotted line in Fig.2(B) is a region where the absorption core 18 is present.

As shown in Figs.2(A) and (B), the front flaps 15a, 15a protruding in the crosswise direction of the diaper are formed in front waist region 15A of the diaper 15. In back waist region 15C, back flaps 15b, 15b are formed, which are present on both the sides of the diaper in the crosswise direction and protrude in the crosswise direction of the back waist region 15C.

The front flaps 15a, 15á and the back flaps 15b, 15b are formed by protruding the back sheet 16 and the top sheet 17 in the crosswise direction of the diaper and bonding the two sheets 16 and 17 together by means of hot-melt adhesive 24, with no absorption core 18 interposed between the back sheet 16 and the top sheet 17. Besides the back sheet 16 and the top sheet 17, alternatively, a resin sheet or a non-woven fabric with a larger base weight is newly cut in a given shape, which is mounted on the back sheet 16 and the top sheet 17 to form front flaps 15a, 15a and back flaps 15b, 15b.

On the surface of the back sheet 16 are fixed one retaining members 19 in the form of retaining fasteners as one example of retaining means, at an interval along the longitudinal direction in the front waist region 15A. Additionally, the other retaining members 20 in the form of retaining fasteners as retaining means are arranged at an interval along the longitudinal direction on the top parts of the back flap 15b (both the sides of the back waist region 15C). When the diaper 15 is worn, the back waist region 15C is applied to the dorsal area of a wearer while the crotch region 15B is applied to the crotch and the front waist region is applied to the abdominal area. Then, the back flaps 15b, 15b are overlaid on the back sheet 16 in the front waist region 15A to fasten the other retaining members 20 with the one retaining members 19.

As shown in Fig.2(A), the interval P2 between the center lines of the widths of two longitudinally aligned other retaining members 20 in the longitudinal direction is larger than the interval P1 between the center lines of the widths of two longitudinally aligned retaining members 19 in the longitudinal direction. The dimension H2 of the ends of the other retaining members 20, 20 in the longitudinal direction is larger than the whole dimension H1 of the ends of the one retaining members 19, 19 in the longitudinal direction. Furthermore, the width dimension B1 of the one retaining members 19 in the longitudinal direction is sufficiently larger than the width dimension B2 of the retaining members 20 in the longitudinal direction.

In the diaper for adults (aged people), for example, the aligning pitch P1 of the one retaining members 19 is 80 mm, while the aligning pitch P2 of the other retaining members 20 is 135 mm. The ratio P2/P1 is almost 3.1/1.9, preferably 3/2 or more. The dimension H1 is 130 mm while H2 is 165 mm, and the difference is 35 mm. Furthermore, the width dimension B1 is 50 mm, while the width dimension B2 is 30 mm, and B1/B2 is preferably 5/3 or more. Furthermore, the one retaining members 19 are of a rectangular shape or a band-like shape, with the longer side toward the crosswise direction of the diaper, and the longer side thereof is for example 235 mm.

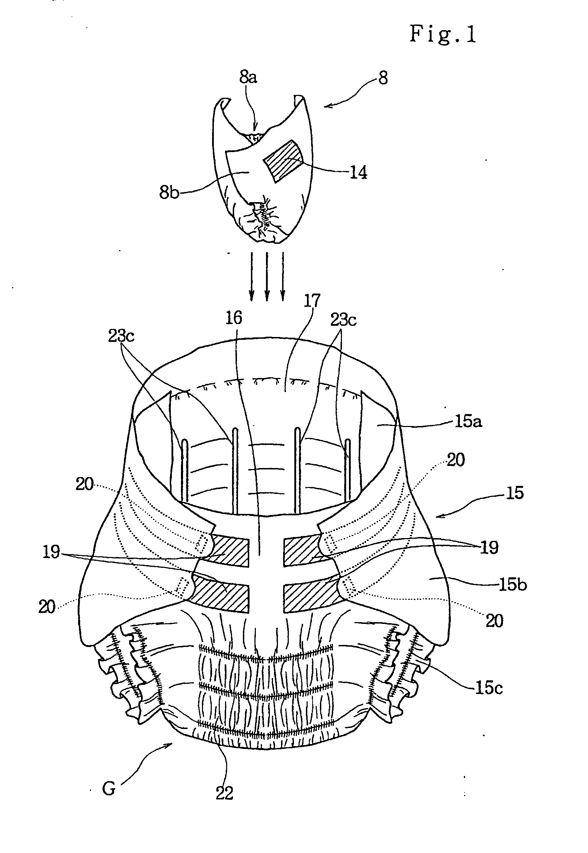

Because the dimension of the one retaining members 19 is large in the crosswise direction, the position to attach these retaining members 19 and 20 together can make a certain shift for wearing, depending on the size of the trunk part of a wearer. Additionally, the aligning pitch P2 of the other retaining members 20 is larger than the aligning pitch P1 of the one retaining members 19, while the dimension H2 of the ends of the other retaining members 20 in the longitudinal direction is larger than the dimension H1 of the ends of the one retaining members 19 in the longitudinal direction. At a state of the diaper worn, therefore, the edge part of the back flap 15b is essentially arranged obliquely toward the longitudinal direction, as shown in Fig.1, while the other retaining members 20, 20 face obliquely toward the longitudinal direction to be fastened on the one retaining members 19.

As shown in Fig.1, accordingly, the position to attach the one retaining members 19 positioned upward in the longitudinal direction of the diaper with the other retaining members 20 is present more inward than the position to attach the one retaining members 19 positioned downward in the longitudinal direction (side of crotch region 15B) with the other retaining members 20. At such state, the other retaining members 20 can be fastened to the one retaining members 19 nearly at the center thereof in the longitudinal direction. Accordingly, at a state of the diaper worn, the hip part of the diaper is large but the trunk part (waist part) thereof is slim, so that the diaper is easy to fit the body and can securely fasten the body around the waist.

In the embodiment, furthermore, the other retaining members 20 are a retaining sheet made of a resin, where a great number of retaining protrusions of a mushroom shape or a hook shape having a retaining head on the top are formed. Alternatively, the one retaining members 19 are woven, tricot-knitted fabric made of a raw material polyester fiber, or non-woven fabric. As the other retaining members 20, an adhesive tape may be arranged satisfactorily instead of the retaining sheet, while instead of woven or non-woven fabric, film may satisfactorily be arranged as the retaining members 19. In this case, adhesive tapes (other retaining members 20) adhere to the surface of film (one retaining members 19) at a state of the diaper worn.

On both the edge parts of the crotch region 15B of the diaper 15, for example, two flat elastic braids 21, 21 at their elongated state in the longitudinal direction and at an interposed state between the back sheet 16 and the top sheet 17 are bonded to the two sheets. The crotch region 15B shrinks in the longitudinal direction via the flat elastic braids 21, 21 to form crinkles to prepare thigh part gathering 15c. Through the thigh part gathering 15c, the diaper turns into a solid shape with the back sheet 16 protruding. When the diaper 15 is worn, the thigh part gathering 15c appropriately fastens the leg groins of a wearer to prevent the forming of a space between the thigh part and the diaper, so that the diaper can fit the thigh part of the wearer.

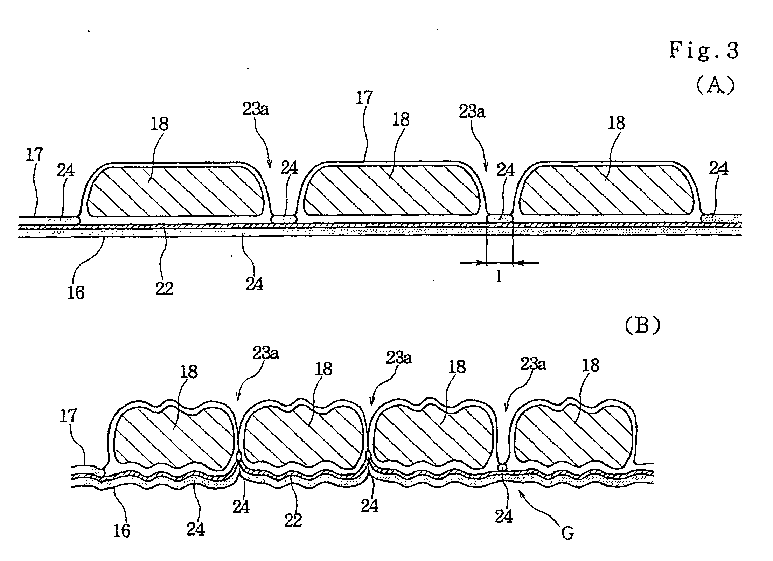

As shown in Fig.2(A), at a part closer to the side of the front waist region from the center part of the crotch region 15B of the diaper 15 in the longitudinal direction, more specifically in region α over the crotch region 15B and the border region between the crotch region 15B and the front waist region 15A, a plurality of elastic members 22 such as flat elastic braid elongating in the crosswise direction of the diaper are arranged at an interval. As shown in Fig. 3 (A), the elastic members 22 are arranged externally on the absorption core 18 and internally on the back sheet 16. Then, the elastic members 22 are bonded and fixed on the back sheet 16 by means of hot-melt adhesive 24 while the elastic members are at an elongated state of a length 1.5-fold to 3-fold, preferably 2-fold to 2.5-fold the initial length. The elastic members 22 and the back sheet 16 are bonded together over the whole length of the elastic members 22 by means of hot-melt adhesive 24. Otherwise, the elastic members 22 and the back sheet 16 are partially bonded together by means of hot-melt adhesive 24. As shown in Fig. 3 (A) , furthermore, the elastic members 22, the top sheet 17 and the back sheet 16 are bonded together and fixed by means of the adhesive 24 in the thin thickness parts 23a.

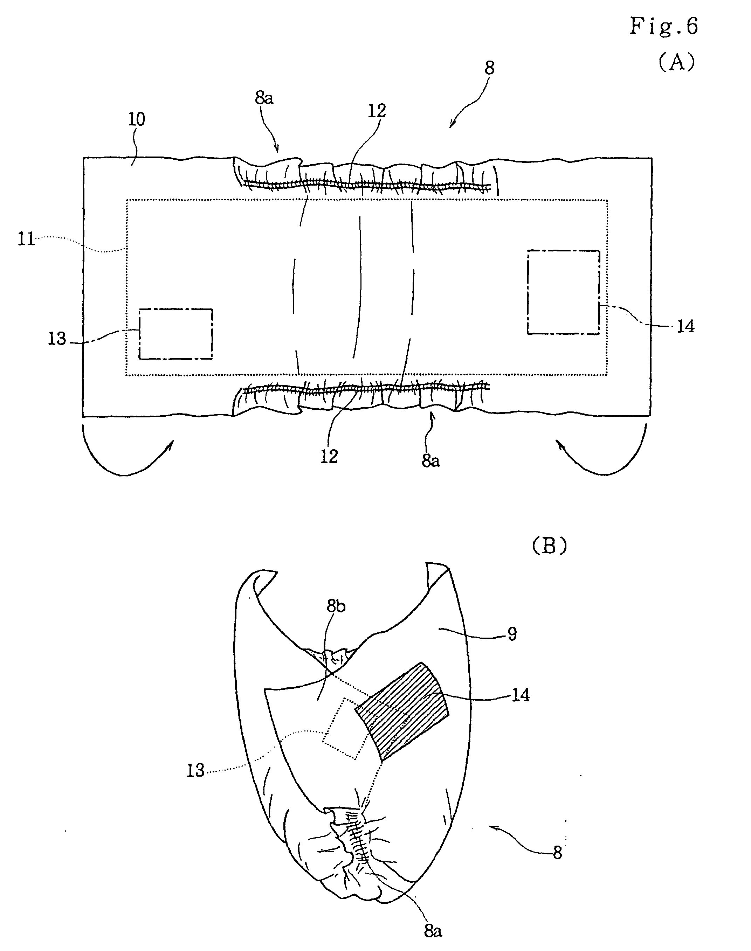

The diaper shrinks in the region α in the crosswise direction through the elastic members 22 to form front gathering G. In one example shown in Fig.2(A), the front gathering G is formed in the crotch region 15B, and the border region between the crotch region 15B and the front waist region 15A. When the diaper is used in combination with protector against incontinence 8 for males as shown in Figs.6(A) and (B), the region α where the elastic members 22 are arranged nearly corresponds to the region where the protector against incontinence 8 is to be mounted. In this case, the distance from the center line O of the crotch region 15B in the crosswise direction to the elastic members 22 arranged at the far end of the front waist region 15A (shown as M in Fig. 2 (A)) is preferably about 250 mm.

As shown in Fig.2 (B), a plurality of thin thickness parts 23a, 23b and 23c are formed on nearly the whole surface of the region where the absorption core 18 is presented on the diaper 15. Thin thickness parts 23a are arranged as three lines elongating linearly in the longitudinal direction in the crotch region 15B and at a given interval in the crosswise direction. Additionally, the thin thickness parts 23b and 23c are arranged as four lines elongating linearly in the longitudinal direction and at a given interval in the crosswise direction. The thin thickness parts 23a arranged in the crotch region 15B, and the thin thickness parts 23b and 23c arranged in the front waist region 15A and the back waist region 15C, are independently formed and separated from each other.

The thin thickness parts 23a arranged in the form of the three lines in the crotch region 15B, and the thin thickness parts 23b arranged in the form of the four lines in the front waist region 15A, are alternately aligned of their end parts in the border region between the crotch region 15B and the front waist region 15A. Therefore, in the parts where the thin thickness parts 23a and 23b are alternately overlaid to each other, the thin thickness parts 23a and 23b are alternately aligned in the crosswise direction of the diaper 15. The parts where the thin thickness parts 23a and 23b are arranged alternately are positioned in the region α where the elastic members 22 are arranged to form front gathering G. It is not required that the part where the thin thickness parts 23a and 23b are alternately aligned accurately corresponds to the border region between the crotch region 15B and the front waist region 15A and the part may reside at a part closer to the side of the crotch region 15B in the border region or at a part closer to the side of the front waist region 15A in the border region. The part where the thin thickness parts 23a and 23b are alternately aligned preferably resides in the region α. However, the part may be more or less outside the region α only if the shrinkage force from the elastic members 22 is given to the part.

In the front waist region 15A, furthermore, the one retaining members 19 elongating in a band-like form in the crosswise direction are arranged at an interval in the longitudinal direction. The thin thickness parts 23b formed in the front waist region 15A elongate to be overlaid on the back side of the region where the one retaining members 19 are arranged.

In the embodiment of the present invention as shown in Figs.3 (A) and (B), no absorption core 18 is present in the thin thickness parts 23a, 23b or 23c. At the thin thickness parts 23a, 23b and 23c, the back sheet 16 and the top sheet 17 are directly bonded together by means of hot-melt adhesive 24 or the like. At the thin thickness parts 23a, 23b and 23c where the elastic members 22 are interposed, preferably, the top sheet 17 and the back sheet 16 are bonded to the elastic members 22.

The thin thickness parts 23a, 23b and 23c elongating linearly in the longitudinal direction are arranged on the diaper 15, and in the region α where the thin thickness parts 23a and 23b are formed, elastic shrinkage force from the elastic members 22 is given to the direction vertical to the thin thickness parts.

When the elastic shrinkage force is larger than the rigidity of the absorption core 18, the parts excluding the thin thickness parts are drawn from both the sides in the crosswise direction of the diaper as shown in Fig.3(B), so that the adjacent parts interposing the thin thickness part 23a adhere together. The parts excluding the thin thickness parts shrink more in the crosswise direction, where the absorption core 18 deforms in the shape of protrusion toward the inside of the diaper (the body side of a wearer). The part of the region α of the diaper 15 adheres to the body, with no forming of any space between the diaper 15 and the body. So as to deform the diaper 15 so that the diaper might fit the body, as has been described above, two or more elastic members may satisfactorily be arranged, each elastic member having an elastic force of 10 g or more when the elastic member elongates to a length two-fold the initial length, provided that the rigidity of the absorption core 18 as measured according to JIS P8125 is 3 g · cm or more to 20 g · cm. Then, the elastic shrinkage force from the elastic members 22 exceeds the rigidity of the absorption core 18, so that the parts excluding the thin thickness parts in the absorption core can shrink. If the elastic shrinkage force of the elastic members 22 is too strong, however, the width dimension of the diaper 15 is too small. The upper limit of the elastic force per one elastic member 22 is about 100 g, while the number of the elastic members 22 varies depending on the elastic force from one elastic member. Generally, however, the number of the elastic members is preferably about two to 50.

In the diaper shown in Fig.2(B), furthermore, both of the end of the thin thickness parts 23a on the side of the crotch region 15B and the end of the thin thickness parts 23b on the side of the front waist region 15A are positioned in the region α under the influence of the elastic force from the elastic members 22. Because both the thin thickness parts 23a and 23b are formed separately from each other, additionally, the number of parts divided by the thin thickness parts 23a and 23b in the absorption core 18 is increased in the border region between the crotch region 15B and the front waist region 15A. Therefore, the diaper readily shrinks at the parts in the crosswise direction, so that the diaper hardly expands forward.

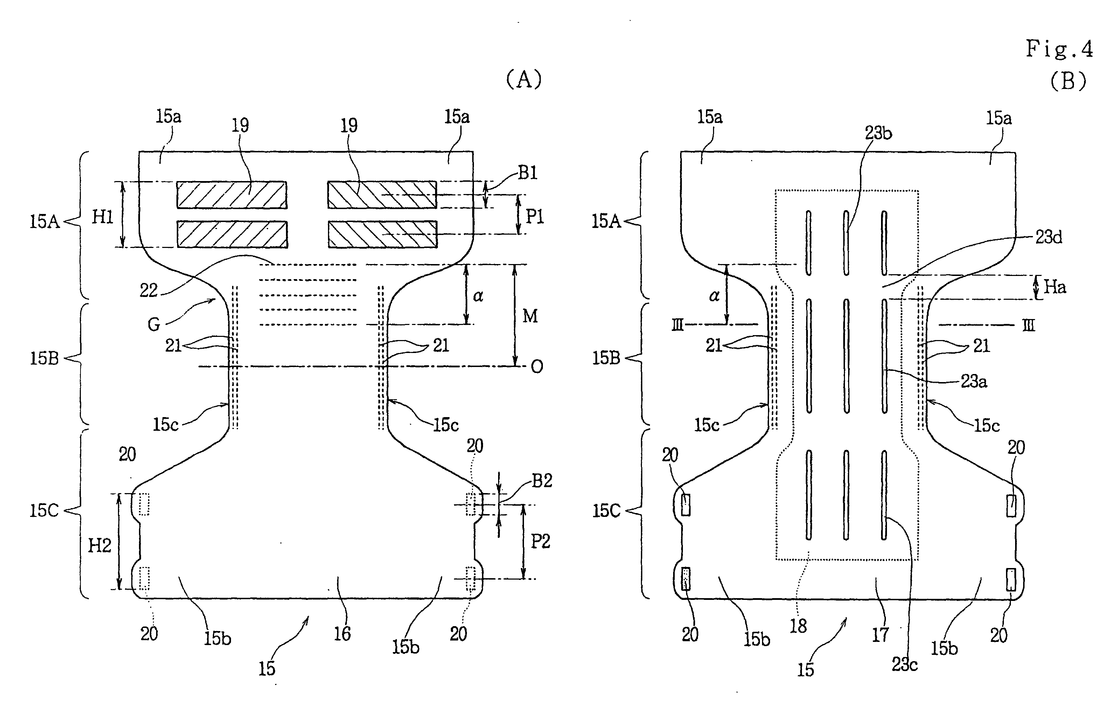

Furthermore, the thin thickness parts 23a and 23b are not necessarily aligned alternately. As long as both the thin thickness parts 23a and 23b are arranged at parts under the influence of the elastic shrinkage force from the elastic members 22, the ends of the two thin thickness parts 23a and 23b may satisfactorily be separated slightly from each other to be present upward and downward in the longitudinal direction, as shown in Fig.4(B). In this case, the crotch region 15B and the front waist region 15A readily shrink in the region α in the crosswise direction, which prevents the forward expansion of the absorption core 18. As shown in Fig.2 (B), however, more satisfactorily, three lines of the thin thickness parts 23a and four lines of the thin thickness parts 23b are alternately arranged in the border region between the crotch region 15B and the front waist region 15A. Because the absorption core 18 is to be divided into eight regions in the crosswise direction at the part, the absorption core 18 under the influence of the shrinkage force from the elastic members 22 is drawn toward the crosswise direction, so that the absorption core more readily shrinks.

Therefore, the diaper 15 fits the body with no forming of any space between the diaper 15 and the body, to prevent the oozing of urine and feces. When the diaper is directly worn, urine is never concentrated at one spot because of the presence of the thin thickness parts 23a of the crotch region 15B, so that urine is readily dispersed over the absorption core in the front waist region 15A and the back waist region 15B.

The thin thickness parts 23a of the crotch region 15B and the thin thickness parts 23b of the front waist region 15A, both receiving the shrinkage force from the elastic members 22, are formed in such a manner that the adjacent thin thickness parts generally have an interval (length L in Fig.2(B)) of about 10 mm or more to 100 mm or less and the width of the thin thickness part 23a or 23b (length l in Fig.2(B)) is 1 mm or more to 15 mm or less. Within the range of such numerical figures, the thin thickness parts 23a and 23b are to be formed. In such case, the diaper 15 readily deforms along the body outline of a wearer, to readily disperse urine.

When a male adult wears the diaper 15, protector against incontinence 8 is generally worn as shown in Figs. 6 (A) and (B) .

The protector against incontinence 8 is formed of a lamination body in a sheet-like form, comprising outer sheet 9 such as liquid non-permeable resin sheet, inner sheet 10 such as non-woven fabric and absorption core 11 interposed between the two sheets. In the regions at the ends of the lamination body in the longitudinal direction and the width direction, where no absorption core is present, the outer sheet and the inner sheet are bonded together by means of a hot-melt adhesive. At the ends in the width direction, elastic members 12 such as flat elastic braids elongating in the longitudinal direction are interposed and partially bonded at its elongated state between the outer sheet and the inner sheet. The elastic members form gathering 8a.

The protector against incontinence 8 is preliminarily formed as a bag-like shape as shown in Fig.6(B), by molding a sheet-like material shown in Fig.6(A) in a conical shape while the outer sheet 9 faces outward. Then, the protector is worn while the overlaying part 8b is applied to the abdominal area of the body. While the protector against incontinence 8 is worn, the back waist region 15C of the diaper 15 is applied to the dorsal area; the crotch region 15B is applied to the crotch and the front waist region 15A is applied to the abdominal area. By adhesion bonding the adhesive part 14 of the protector against incontinence 8 to the top sheet 17 of the front waist region and then fastening the one retaining members 19 and 20 together, the diaper 15 is worn. Then, the diaper 15 deforms through the thin thickness parts 23a, 23b and 23c along the body outline of the body of the wearer, and furthermore, the region α in the border between the front waist region 15A and the crotch region 15B deforms along the shape of the protector against incontinence 8. Subsequently, the front gathering G as applied to the crotch of the wearer elongates along the outer shape of the protector against incontinence 8, to elastically fit the protector against incontinence 8, whereby the protector against incontinence 8 can be pressed so that the protector might never be free from the body of the wearer. Thus, the protector against incontinence 8 does not make any shift in position, so that the protector against incontinence 8 effectively absorbs urine.

When the diaper 15 is used for females, additionally, the protector against incontinence 8 is overlaid on the top sheet 17 of the crotch region 15B of the diaper 15 while the protector against incontinence 8 remains as the sheet-like form as shown in Fig. 6 (A) , and at such state, the diaper 15 is worn. The front gathering G is in contact to the protector against incontinence 8, so that the protector against incontinence 8 can be retained at a stable state by means of the front gathering G. When the protector against incontinence 8 is used for females, preferably, elastic members 22 are arranged more closely to the side of the center line O as shown in Fig.2(A).

When the protector against incontinence 8 is used in such manner, the diaper worn expands between the crotch and hip parts. As has been described above, however, the other retaining members 20, 20 at their oblique state in the longitudinal direction are fastened to the one retaining members 19, 19 in the diaper. Therefore, even the diaper with an expansion formed as described above can have a slimmer waist part to securely fasten the retaining members 19, 20 around the waist of the wearer.

When the diaper 15 is singly worn with no use of any protector against incontinence, the front gathering G deforms along the shape of the crotch of a wearer to elastically fit the crotch. Thus, no space is formed between the body of the wearer and the diaper 15, so that the side oozing of urine and feces from the diaper can be prevented, whereby urine and feces can be retained securely on the diaper 15. Through the thin thickness parts 23a, 23b and 23c, urine can be dispersed not only over the crotch region, without any concentration, so that the absorption potency of the diaper 15 can be improved.

As shown in Figs.2(A) and (B), furthermore, the thin thickness parts 23b formed in the front waist region 15A elongate to a position where the thin thickness parts 23b are overlaid over the one retaining members 19. When the back flaps 15b, 15b are overlaid on the front waist region 15A to fasten the retaining members 19, 20 together, the surface of the front waist region 15A readily deforms. If the front gathering G is formed around the part where the one retaining members 19 are arranged owing to the presence of the elastic members 22 as shown in Fig. 2 (A), in particular, the shrinkage force of the elastic members 22 acts on a part of the one retaining members 19 so that the one retaining members 19 readily receive the shrinkage force in the crosswise direction.

At a part where the one retaining members 19 are arranged, the width of the thin thickness part 23b is narrowed similarly as shown in Fig.3(A), because of the shrinkage force, but at a part where the absorption core is present between the thin thickness part 23b and the thin thickness part 23b, the thin thickness parts 23b slightly shrink in the crosswise direction but the surface is still flat. At the one retaining members 19, therefore, the part between the thin thickness parts 23b, 23b is at a flat state and the area of the flat part is relatively large. Hence, the other retaining members 20 can be bonded to the flat part of the one retaining members 19. Thus, the retaining strength between the retaining members 19, 20 can be kept large.

As has been described above, in Figs. 4 (A) and (B), the end parts of the thin thickness parts 23a in the crotch region 15B and of the thin thickness parts 23b in the front waist region 15A are more or less separated from each other in the longitudinal direction, and the region of a given width Ha between the thin thickness parts 23a and 23b in the longitudinal direction corresponds to thick thickness part 23d with no thin thickness part formed thereon. The thick thickness part 23d is positioned in the region α where the elastic members 22 are arranged to form front gathering G.

As shown in Fig.3(B), due to the shrinkage of the elastic members 22 in the crosswise direction, recesses are formed at the part where the thin thickness parts 23a or 23b are formed. Therefore, recess is formed on the top sheet 17, to make the irregularity of the top sheet 17 more prominent. When the thick thickness part 23d is arranged, however, the irregularity of the top sheet 17 is slightly reduced at the thick thickness part 23d, to consequently enlarge the relatively flat area. When the protector against incontinence 8 is positioned inside the front gathering G and the adhesive part 14 adheres to the thick thickness part 23d, the area where the adhesive part 14 and the top sheet 17 face to each other can be enlarged because no thin thickness part is present. Thus, the adhesion strength of the protector against incontinence 8 to the diaper can be enhanced, whereby the shift of the protector against incontinence 8 can be prevented.

As has been described above, additionally, the thin thickness parts 23a and 23b are present in front of and behind the thick thickness part 23d. Thus, it never occurs that the diaper 15 hardly shrinks due to the presence of the thick thickness part 23d.

The thick thickness part 23d is formed at a part to be spotted with urine. Because a greater volume of the absorption core 18 is present at the thick thickness part 23d, the reduction of the absorbed urine in volume can be prevented.

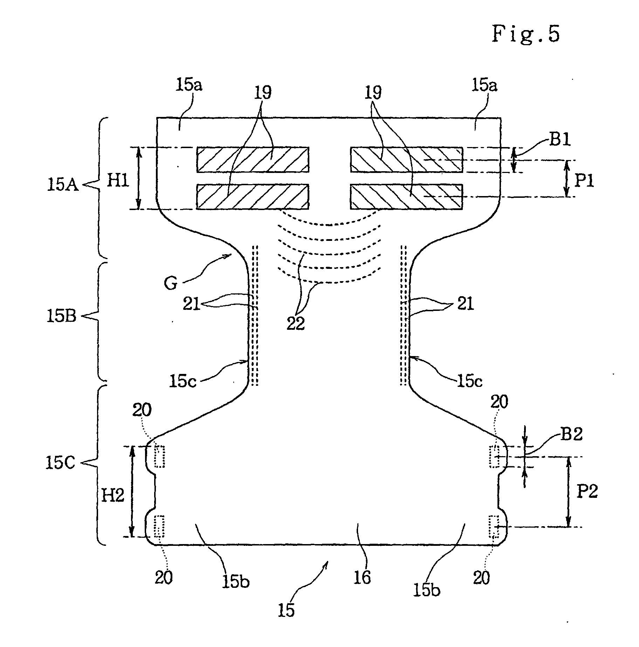

Fig.5 depicts another embodiment of the diaper of the present invention, which is a view of the diaper 15 at a developed state, as viewed from the outside. In the embodiment, elastic members 22 are arranged with a curve protruding toward the center direction of the crotch region 15B of the diaper 15, from both the ends of the diaper 15 to the center thereof. If the elastic members 22a are arranged in such fashion, the diaper 15 deforms more fittingly along the protector against incontinence 8 and the crotch shape of a wearer, so that the diaper 15 can fit the body more.

Even in Fig.5, furthermore, the aligning pitch P2 of the other retaining members 20 in the longitudinal direction is larger than the aligning pitch P1 of the one retaining members 19 in the longitudinal direction. Additionally, the dimension H2 between the end parts of the other retaining members 20 is larger than the dimension H1 between the end parts of the one retaining members 19. Still further, the width dimension B1 of the one retaining members 19 in the longitudinal direction is sufficiently larger than the width dimension B2 of the other retaining members 20 in the longitudinal direction.

When the diaper 15 is worn on the body, the trunk part of the diaper 15 can essentially be slimmed to securely fasten the diaper 15 around the waist part of wearer.

Even in Fig.5, the thin thickness parts 23b are formed in a region to be overlaid on the one retaining members 19 in the front waist region 15A.

The present invention has been described in the examples of an open type diaper, but the effect of arranging the thin thickness parts 23a, 23b and 23c and the effect of forming the front gathering G, in accordance with the present invention, are also exerted even if the thin thickness parts are formed in a brief-type diaper. Such thin thickness parts may satisfactorily be formed in diapers not only for adults but also for infants (and toddlers).

As has been described in detail insofar, in the diaper of the present invention, elastic members are arranged in the front waist region or the border region between the crotch region and the front waist region to form gathering. In the region where the gathering is formed, the.crotch of a wearer can be pressed elastically. Thus, no space is formed between the diaper and the crotch of a wearer, so that the diaper can fit the crotch.

Because the thin thickness parts elongating in the longitudinal direction of the diaper are formed, the diaper can readily deform along the body outline of the wearer owing to the presence of the thin thickness parts. And the absorption core shrinks in the crosswise direction to effectively prevent the expansion of the crotch region in a bag-like form. Furthermore, the thin thickness parts formed in the crotch region elongate into the region between the thin thickness parts in the front waist region and in the border region between the front waist region, and the crotch region, the thin thickness parts in the front waist region and the thin thickness parts in the crotch region are alternately arranged. Owing to the presence of the gathering formed in the region the diaper shrinks in the crosswise direction but hardly expands forward, so that the diaper deforms into a shape adjusting to the crotch of a wearer. Through the gathering, the diaper can fit the crotch of the wearer in such manner.

When the diaper is used in combination with a protector against incontinence, therefore, the diaper does not shift the protector against incontinence in position to securely retain the protector against incontinence and absorb urine effectively owing to the presence of the protector against incontinence. Even when the diaper is used singly with no use of any protector against incontinence, the diaper still can retain excretion such as urine and feces securely, without causing any side oozing thereof.

Because the elastic members are arranged only at the part where the protector against incontinence is mounted, additionally, the parts excluding the aforementioned part can touch skin at the natural state, which gives pleasant feeling to the whole body when the diaper is worn.

By determining relatively the positions of the one retaining members and the other retaining members, additionally, the diaper can be made broad around the hip part and be made slim around the trunk part when the diaper is worn. Thus, the diaper can be fastened securely around the trunk part of a wearer, whereby the positional shift of the diaper hardly occurs. Even if the diaper expands in the crotch or at the hip part, the diaper can securely fasten the trunk part of the wearer so that the diaper can readily fit the body shape of the wearer.

## Claims

1. A disposable diaper comprising a liquid non-permeable back sheet (16), a liquid permeable top sheet (17) and an absorption core (18) interposed between the two sheets, having a front waist region (15A) to be applied to the abdominal area of a wearer, a back waist region (15C) to be applied to the dorsal area thereof, and a crotch region (15B) to be applied to the crotch, wherein thin thickness parts (23A, 23B, 23C) in the absorption core where either no absorption core is present or where the thickness of the absorption core is reduced are formed in a linearly elongating fashion in the longitudinal direction at least in the crotch region, provided that the direction from the front waist region through the crotch region to the back waist region is defined as the longitudinal direction; wherein elastic members (22) are arranged to give a shrinkage force to the absorption core toward the direction of crossing the thin thickness parts; **characterised in that** the elastic members (22) are arranged on the side of the absorption core facing the backsheet and are arranged only in a region (a) of the absorbent core which extends only over a part of the front waist region (15A) and an adjacent part of the crotch region (15B) which is located between the centre (o) of the diaper in the longitudinal direction and the front waist region (15A).

2. A disposable diaper according to claim 1, wherein the thin thickness parts (23A) formed in the crotch region and the thin thickness parts (23B) formed in the front waist region are alternately aligned around the border between the crotch region and the front waist region and wherein the elastic members (22) give a shrinkage force to the absorption core at the parts where the thin thickness parts are alternately aligned.

3. A disposable diaper according to claim 1, wherein one retaining member(s) (1A) is (are) arranged on the surface of the front waist region to overlay and fasten the
back waist region and wherein the thin thickness parts (23B) elongate into the region where the one retaining member is arranged.

4. A disposable diaper according to claim 1, wherein the elastic members (22) are arranged between the absorption core (18) and the back sheets (16) and wherein the elastic members (22), the top sheet (17) and the back sheet (16) are bonded together at the thin thickness parts.

5. A disposable diaper according to claim 1, wherein the elastic members (22) are arranged with a curve toward the centre of the crotch region in the longitudinal direction at a developed state of the diaper on a plane.

6. A disposable diaper according to claim 1, wherein the thin thickness parts (23B) arranged in the front waist region and the thin thickness parts (23A) arranged in the crotch region are independently and separately formed from each other at a position closer to the side of the front waist region from the centre of the longitudinal direction and wherein thick thickness part(s) where no thin thickness part is present is formed at parts between the ends of the thin thickness parts (23A, 23B) in the longitudinal direction.

7. A disposable diaper according to claim 6, wherein the elastic members (22) are arranged in the region including the thick thickness part(s) and a protector (8) against incontinence is mounted in the region including the thick thickness part(s) when the protector against incontinence is mounted on the interior side of the top sheet for use.

## Patentansprüche

1. Wegwerfwindel, die eine flüssigkeitsundurchlässige Grundschicht (16), eine flüssigkeitsdurchlässige Deckschicht (17) und einen zwischen den zwei Schichten angeordneten absorbierenden Kern (18) umfasst, die eine vordere Taillenregion (15A), die an den abdominalen Bereich eines Trägers anzubringen ist, eine hintere Taillenregion (15C), die an den dorsalen Bereich davon anzubringen ist und eine Schrittregion (15B), die an den Schritt anzubringen ist, aufweist, worin die Teile dünner Dicke (23A, 23B, 23C) im Absorptionskern, wo entweder kein Absorptionskern vorhanden ist, oder wo die Dicke des Absorptionskerns reduziert ist, in einer sich linear verlängernden Weise in der longitudinalen Richtung wenigstens in der Schrittregion geformt sind, vorausgesetzt, dass die Richtung von der vorderen Taillenregion durch die Schrittregion zur hinteren Taillenregion als die longitudinale Richtung definiert ist;
worin elastische Elemente (22) so angeordnet sind, dass sie dem Absorptionskern eine Schrumpfkraft in Richtung des Übergangs der Teile dünner Dicke geben;
**dadurch gekennzeichnet, dass** die elastischen Elemente (22) auf der Seite des Absorptionskerns angeordnet sind, die der Grundschicht gegenüberliegt und nur in einer Region (a) des absorbierenden Kerns angeordnet sind, der sich nur über einen Teil der vorderen Taillenregion (15A) und einen benachbarten Teil der Schrittregion (15B) erstreckt, der zwischen dem Zentrum (o) der Windel in der Längsrichtung und der vorderen Taillenregion (15A) liegt.

2. Wegwerfwindel nach Anspruch 1, worin die in der Schrittregion geformten Teile dünner Dicke (23A) und die in der vorderen Taillenregion geformten Teile dünner Dicke (23B) abwechselnd um die Grenze zwischen der Schrittregion und der vorderen Taillenregion ausgerichtet sind, und worin die elastischen Elemente (22) dem Absorptionskern eine Schrumpfkraft an den Teilen geben wo die Teile dünner Dicke abwechselnd ausgerichtet sind.

3. Wegwerfwindel nach Anspruch 1, worin ein Halteelement (e) (1A) an der Oberfläche der vorderen Taillenregion angeordnet ist (sind), um die hintere Taillenregion zu bedecken und zu befestigen, und worin sich die Teile dünner Dicke (23B) in die Region verlängern wo das eine Halteelement angeordnet ist.

4. Wegwerfwindel nach Anspruch 1, worin die elastischen Elemente (22) zwischen dem Absorptionskern (18) und den Grundschichten (16) angeordnet sind, und worin die elastischen Elemente (22), die Deckschicht (17) und die Grundschicht (16) an den Teilen dünner Dicke miteinander verbunden sind.

5. Wegwerfwindel nach Anspruch 1, worin die elastischen Elemente (22) kurvenförmig in Richtung der Mitte der Schrittregion in der Längsrichtung in einem entwickelten Zustand der Windel auf einer Ebene angeordnet sind.

6. Wegwerfspindel nach Anspruch 1, worin die in der vorderen Taillenregion angeordneten Teile dünner Dicke (23B) und die in der Schrittregion angeordneten Teile dünner Dicke (23A) an einer Stelle, die der Seite der vorderen Taillenregion ab dem Zentrum der longitudinalen Richtung näher liegt, unabhängig und getrennt voneinander geformt sind, und worin das (die) Teil (e) dicker Dicke wo kein Teil dünner Dicke vorhanden ist, an Teilen zwischen den Enden der Teile dünner Dicke (23A, 238) in der Längsrichtung geformt ist.

7. Wegwerfwindel nach Anspruch 6, worin die elastischen Elemente (22) in der Region angeordnet sind, die das (die) Teil(e) dicker Dicke einschließt, und ein Schutz (8) gegen Inkontinenz in der Region montiert ist, die das (die) Teil (e) dicker Dicke einschließt, wenn der Schutz gegen Inkontinenz zum Gebrauch auf der Innenseite der Deckschicht montiert ist.

## Revendications

1. Couche-culotte jetable comprenant une feuille arrière non perméable aux liquides (16), une feuille supérieure perméable aux liquides (17) et un coeur d'absorption (18) interposé entre les deux feuilles, avec une zone de ceinture avant (15A) destinée à être appliquée à la partie abdominale d'un porteur, une zone de ceinture arrière (15C) destinée à être appliquée à la partie dorsale de celui-ci, et une zone d'entre-jambes (15B) destinée à être appliquée à l'entre-jambes, dans laquelle des parties d'épaisseur fine (23A, 23B, 23C) dans le coeur d'absorption, dans lesquelles soit aucun coeur d'absorption n'est présent soit l'épaisseur du coeur d'absorption est limitée, sont formées d'une façon allongée linéairement dans la direction longitudinale, au moins dans la zone d'entre-jambes, étant entendu que la direction depuis la zone de ceinture avant à travers la zone d'entre-jambes jusqu'à la zone de ceinture arrière est définie comme la direction longitudinale ; dans laquelle des éléments élastiques (22) sont agencés pour procurer une force de rétrécissement au coeur d'absorption vers la direction de croisement des parties d'épaisseur fine ; **caractérisée en ce que** les éléments élastiques (22) sont agencés sur la face du coeur d'absorption tournée vers la feuille arrière, et sont agencés uniquement dans une zone (α) du coeur d'absorption qui s'étend uniquement sur une partie de la zone de ceinture avant (15A) et une partie adjacente de la zone d'entre-jambes (15B) qui est située entre le centre (o) de la couche-culotte dans la direction longitudinale et la zone de ceinture avant (15A).

2. Couche-culotte jetable selon la revendication 1, dans laquelle les parties d'épaisseur fine (23A) formées dans la zone d'entre-jambes et les parties d'épaisseur fine (23B) formées dans la zone de ceinture avant sont alignées alternativement autour de la bordure entre la zone d'entrejambes et la zone de ceinture avant, et dans laquelle les éléments élastiques (22) procurent une force de rétrécissement au coeur d'absorption sur les parties où les parties d'épaisseur fine sont alignées alternativement.

3. Couche-culotte jetable selon la revendication 1, dans laquelle un ou des élément(s) de rétention (1A) est(sont) agencé(s) sur la surface de la zone de ceinture avant pour recouvrir et fixer la zone de ceinture arrière, et dans laquelle les parties d'épaisseur fine (23B) se prolongent dans la zone où l'élément de rétention est agencé.

4. Couche-culotte jetable selon la revendication 1, dans laquelle les éléments élastiques (22) sont agencés entre le coeur d'absorption (18) et les feuilles arrière (16) et dans laquelle les éléments élastiques (22), la feuille supérieure (17) et la feuille arrière (16), sont collés les uns aux autres au niveau des parties d'épaisseur fine.

5. Couche-culotte jetable selon la revendication 1, dans laquelle les éléments élastiques (22) sont agencés avec une courbure vers le centre de la zone d'entre-jambes dans la direction longitudinale, dans un état développé de la couche-culotte sur un plan.

6. Couche-culotte jetable selon la revendication 1, dans laquelle les parties d'épaisseur fine (23B) agencées dans la zone de ceinture avant et les parties d'épaisseur fine (23A) agencées dans la zone d'entre-jambes sont formées de manière indépendante et séparées les unes des autres à un emplacement plus proche du côté de la zone de ceinture avant depuis le centre de la direction longitudinale, et dans laquelle une ou des partie(s) d'épaisseur fine où aucune partie d'épaisseur fine n'est présente est (sont) formée(s) sur des parties situées entre les extrémités des parties d'épaisseur fine (23A, 23B) dans la direction longitudinale.

7. Couche-culotte jetable selon la revendication 6, dans laquelle les éléments élastiques (22) sont agencés dans la zone comprenant la ou les partie(s) d'épaisseur fine, et une protection (8) contre l'incontinence est placée dans la zone comprenant la ou les partie(s) d'épaisseur fine lorsque la protection contre l'incontinence est montée sur la face intérieure de la feuille supérieure pour son utilisation.
